# EUROPÄISCHE PATENTSCHRIFT

(11) **EP 0 895 459 B1**
(45) Veröffentlichungstag und Bekanntmachung des Hinweises auf die Patenterteilung: **14.11.2001**
(21) Anmeldenummer: 97922864.0
(22) Anmeldetag: 25.04.1997
(51) Int. Cl.: A61B 17/02, A61B 1/12

(54) **CHIRURGISCHES INSTRUMENTENSYSTEM**
SURGICAL INSTRUMENT SYSTEM
SYSTEME D'INSTRUMENT CHIRURGICAL

(30) Priorität: 25.04.1996 DE 19616610
(43) Veröffentlichungstag der Anmeldung: 10.02.1999
(73) Patentinhaber: Karl Storz GmbH & Co. KG, 78532 Tuttlingen (DE)
(72) Erfinder: MANHES, Hubert, F-03200 Vichy (FR)
(74) Vertreter: Münich, Wilhelm, Dr.
(86) Internationale Anmeldenummer: DE9700839
(87) Internationale Veröffentlichungsnummer: WO9740750

(56) Entgegenhaltungen:
- EP-A- 0 537 573
- WO-A-94/11052
- WO-A-95/10982
- FR-A- 2 639 819
- GB-A- 2 161 725

## Beschreibung

### Technisches Gebiet

Die Erfindung bezieht sich auf ein chirurgisches Instrumentensystem insbesondere zur Verwendung bei nicht-endoskopischen Operationen.

### Stand der Technik

Bei endoskopischen Operationen werden die Instrumente durch vorhandene oder künstlich geschaffene Zugänge in natürliche Körperhöhlen eingesetzt. Damit ist es möglich, mit einem Gas, wie beispielsweise CO₂ die Körperhöhle während des Operationsvorganges "aufzublasen" und so den nötigen "Arbeitsraum" für den Operationsvorgang zu schaffen bzw. aufrechtzuerhalten.

Bei herkömmlichen nicht-endoskopischen" Operationen, also bei Operationen, bei denen der menschliche Körper geöffnet und nicht in einer natürlichen Körperhöhle gearbeitet wird, werden häufig anatomisch vorgegebene Schnittstellen benutzt, um einen "Zugang" in den Körper zu schaffen, wobei bevorzugt längs sogenannter Spaltungs- bzw. Schnittebenen gearbeitet wird Im Gegensatz zu den Körperhöhlen stehen die verschiedenen möglichen Spaltungsebenen untereinander in Verbindung, so daß es insbesondere unmöglich ist, ein unter Druck stehendes Gas zur Aufweitung und zur Spülung des unmittelbar das Instrument umgebenden Bereichs zu verwenden.

Ein Problem bei Operationen ist u.a., das (Operations)-Instrument schonend längs derartiger natürlicher Schnitt- bzw. Spaltungsebenen im Körper vorwärts zu bewegen.

Unter Schnitt- bzw. Spaltungsebenen werden im Rahmen der vorliegenden Beschreibung beispielsweise Flächen verstanden, an denen unterschiedliche Körper- bzw. Gewebeschichten, wie beispielsweise verschiedene Muskelgewebe(Schichten) oder Muskel- und Fettgewebe oder Fettgewebe und Adern aneinander angrenzen. Allgemeiner ausgedrückt, sind derartige Ebenen die Grenzflächen zwischen unterschiedlichen Bereichen, von denen jeder insbesondere homogen sein kann.

### Darstellung der Erfindung

Der Erfindung liegt die Aufgabe zugrunde, ein chirurgisches Instrumentensystem, das in den menschlichen oder tierischen Körper insbesondere bei herkömmlichen, nicht-endoskopischen Operationen eingesetzt wird, zu schaffen, das in einfacher Weise längs natürlicher Schnitt- bzw. Spaltungsebenen vorwärts bewegt werden kann.

Eine erfindungsgemäße Lösung dieser Aufgabe ist im Patentanspruch 1 angegeben. Weiterbildungen der Erfindung sind Gegenstand der Ansprüche 2 folgende.

Die Erfindung geht von der Erkenntnis aus, daß Wasser, gegebenenfalls mit gelösten physiologischen Stoffen, wie Salzen etc. ein ideales Distensionselement zur Aufweitung bzw. Spaltung des Körpergewebes längs Grenzflächen bzw. Spaltungsebenen ist.

Aus der WO-A-9510982 ist ein chirurgisches Instrument für subkutane endoskopische Eingriffe bekannt. Dieses Instrument weist einen Schaft auf, der einen Zufuhrkanal mit einer distal angeordneten Fluid-Austrittsöffnung umfasst. Dieses Instrument ist nicht dazu geeignet, ein Instrumentensystem bereitzustellen, dass in den menschlichen oder tierischen Körper einsetzbar ist und insbesondere bei konventionellen chirurgischen Eingriffen in einfacher Weise längs natürlicher Schnitt- bzw. Spaltungsebenen vorwärts bewegt werden kann.
Sowohl das in der WO-A-9510982 beschriebene Instrument als auch alle anderen aus dem Stand der Technik bekannten Instrument stellen im wesentlichen Endoskope dar, die natürliche Körpergänge mittels Gaszufuhr erweitern oder zum Einführen die Herstellung einer künstlichen Öffnung erfordern.

Erfindungsgemäß wird deshalb nach dem Öffnen des Körpers, d.h. nach dem Schaffen eines Zugangs ein Operationsinstrument eingesetzt, aus dessen distalem Ende ein Flüssigkeitsstrahl austritt, der das Gewebe "aufweitet" bzw. "spaltet" , so daß sich eine Flüssigkeits- bzw. "Wasserblase" mitten in natürliche Schnittebenen vorwärts bewegen kann, die den Vorschub des Operationsinstruments insbesondere ohne Durchtrennung von homogenen Gewebebereichen ermöglicht.

Die zugeführte Flüssigkeit wird mit einer derartigen Saugleistung wieder abgepumpt, daß sich (bevorzugt) lediglich eine Flüssigkeitsblase bildet, die das distale Ende des Operationsinstruments umgibt. Damit kann das Instrument längs einer natürlichen Schnitt- bzw. Spaltungsebene vorwärts bewegt werden, wobei die Spaltungsebene durch die mit Druck austretende Flüssigkeit wenigstens in dem Maße geöffnet wird, daß das Operationsinstrument eingesetzt und vorwärts bewegt werden kann. Andererseits wird ein zu großer Flüssigkeitseintrag, der beispielsweise zu einer "Überschwemmung des Operationsfeldes führen würde, durch das Abpumpen von Flüssigkeit aus der Flüssigkeitsblase vermieden.

Das erfindungsgemäße chirurgische Instrumentensystem, das insbesondere zur Verwendung bei nicht-endoskopischen Operationen ausgelegt ist, weist deshalb
- ein Operationsinstrument, das einen Schaft aufweist, in dem ein Zufuhrkanal mit einer distal angeordneten Fluid-Austrittsöffnung, und ein Absaugkanal mit einer distal angeordneten Fluid-Ansaugöffnung vorgesehen sind, und
- eine Pumpeneinrichtung auf, die eine Flüssigkeit mit einem derartigen Druck in den Zufuhrkanal pumpt, daß das Gewebe vor dem distalen Ende des Operationsinstruments durch die aus der Austrittsöffnung austretende Flüssigkeit längs natürlicher Spaltungsebenen getrennt wird, und die die Flüssigkeit aus der das distale Ende des Operationsinstruments umgebenden Flüssigkeitsblase durch den Absaugkanal zum proximalen Ende des Operationsinstruments pumpt.

Bevorzugt kann in dem Schaft wenigstens ein - insbesondere zentral bzw. mittig angeordneter - Operationskanal vorgesehen sein, der das Einführen von Behandlungsinstrumenten, wie z.B. Instrumenten zum Schneiden, Spreizen oder Koagulieren ermöglicht. Damit kann das Instrument nicht nur längs Spaltungsebenen bewegt werden, sondern es ist auch die Durchführung von Eingriffen ohne Instrumentenwechsel möglich.

Um das Vorwärtsschieben des Instruments sowie die Durchführung des eigentlichen Operationsvorgangs beobachten zu können, ist es ferner bevorzugt, wenn der Schaft eine Endoskopoptik aufweist, oder wenn der Schaft einen Kanal aufweist, in den eine Endoskopoptik einsetzbar ist. Um insbesondere bei einer außermittigen Anordnung der Endoskopoptik das Vorschieben des Instruments überwachen zu können, ist es von Vorteil, wenn das Objektiv der Endoskopoptik eine Blickrichtung bzw. eine objektseitige optische Achse hat, die einen kleinen Winkel, der z.B. ca. 12° betragen kann, mit der Längsachse des Schaftes einschließt.

Als "Schaft-Operationsinstrumente" können im Prinzip die verschiedensten Instrumente eingesetzt werden, wie sie insbesondere aus der Endoskopie bekannt sind.

Besonders bevorzugt ist es jedoch, wenn der Schaft den Grundaufbau einer Laparoskops mit seitlich versetztem Einblick bzw. Okular hat, so dass in dem Laparoskop ein durchgehender Kanal vorhanden ist. In diesen Kanal wird erfindungsgemäß eine Doppelkanüle eingesetzt, in der der Zufuhrkanal und der Absaugkanal vorgesehen sind. Weiterhin kann auch der Operationskanal - bevorzugt zentral - in der Doppelkanüle vorgesehen sein. Durch die zentrale Anordnung des Operationskanals ergibt sich eine vorteilhafte Umspülung des in den Operationskanal eingesetzten Instruments.

Bei der Verwendung einer Doppelkanüle, in der der Zufuhrkanal und der Absaugkanal vorgesehen sind, ist es weiterhin bevorzugt, wenn diese die beiden Anschlüsse für die Pumpeneinrichtung aufweist. Diese Anschlüsse können in der unterschiedlichsten Art ausgebildet und beispielsweise Luer-Lock-Anschlüsse sein.

Die Pumpeneinrichtung, die beispielsweise eine (Sero)-Konditionierpumpe nach Manhes sein kann, kann insbesondere von einer elektronischen Steuereinheit gesteuert werden. Diese steuert bzw. regelt den Druck, mit dem die Flüssigkeit austritt, derart, dass er der Konsistenz bzw. der Dichte der zu trennenden Gewebeschichten etc. angepasst ist. Bei einer Reihe von Anwendungsfällen ist es bevorzugt, wenn der Druck, mit dem die Flüssigkeit austritt, zwischen ca. 10⁴ Pascal (0,1 bar) und ca. 10⁵ Pascal (1 bar) liegt.

### Kurze Beschreibung der Zeichnung

Die Erfindung wird nachstehend ohne Beschränkung des allgemeinen Erfindungsgedankens anhand von Ausführungsbeispielen unter Bezugnahme auf die Zeichnung exemplarisch beschrieben, auf die im übrigen hinsichtlich der Offenbarung aller im Text nicht näher erläuterten erfindungsgemäßen Einzelheiten ausdrücklich verwiesen wird.
Es zeigen:
- Fig. 1: eine Seitenansicht eines erfindungsgemäß verwendeten Instruments, und
- Fig. 2: eine erfindungsgemäß ausgebildete Doppelkanüle.

### Darstellung eines Ausführungsbeispiels

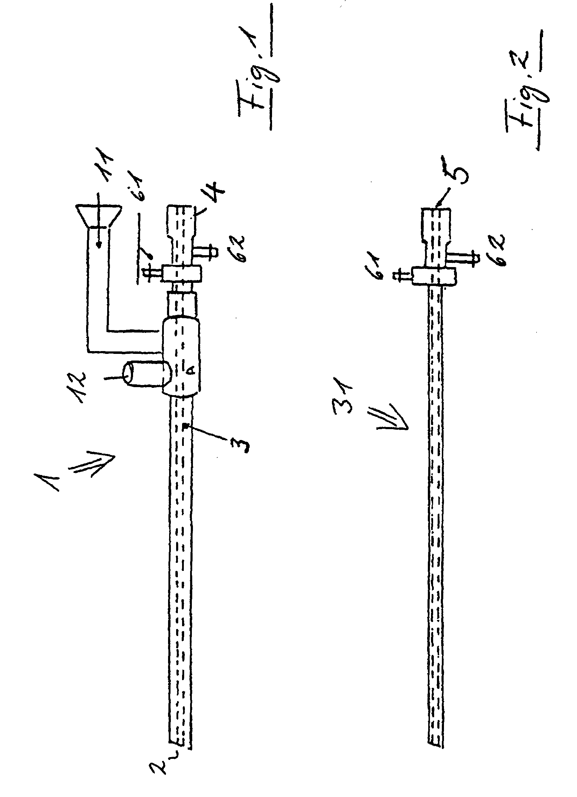

Fig. 1 zeigt ein Instrument, das erfindungsgemäß als "Schaft-Operationsinstrument" verwendet werden kann.

Dieses Instrument hat den Grundaufbau eines an sich bekannten Laparoskops 1 mit seitlichem Einblick bzw. Okular 11 und mit einem Anschluß 12 für ein Lichtleitkabel, durch das das Instrument mit einer nicht dargestellten Lichtquelle verbunden werden kann. Das Licht der Lichtquelle beleuchtet das vor dem distalen Ende 2 des Laparoskops 1 liegende Objektfeld, das mittels eines - nicht dargestellten - Objektivs, ebenfalls nicht dargestellter Bildweiterleiter, die insbesondere Stablinsen-Relaissysteme oder Bildfaserbündel enthalten können, und des Okulars 11 betrachtet werden kann. An das Okular 11 kann in bekannter Weise eine Videoeinheit angeflanscht werden.

Derartige Laparoskope werden beispielsweise von der Karl Storz GmbH & Co., Tuttlingen, Deutschland hergestellt. Auf den Aufbau dieser Instrumente wird zur Erläuterung aller hier nicht näher beschriebenen Einzelheiten ausdrücklich verwiesen.

Bei dem gezeigten Ausführungsbeispiel weist das Instrument ein Endoskopobjektiv mit schrägstehender objektseitiger optischer Achse auf. Die objektseitige optische Achse schließt bevorzugt einen Winkel von ca. 12° gegenüber der Instrumentenachse A ein.

In an sich bekannter Weise weist das Laparoskop 1 ferner einen insbesondere zentral angeordneten durchgehenden Kanal 3 auf, der das distale Ende 2 mit dem proximalen Ende 4 des Instruments 1 verbindet, und in den weitere Instrumente eingesetzt werden können.

Erfindungsgemäß ist in den Kanal 3 eine spezielle Kanüle 31 eingesetzt, die in Fig. 2 näher dargestellt ist, und die nachstehend beschrieben wird.

Die in Fig. 2 näher dargestellte Kanüle 31 wird in das Laparoskop 1 eingeschoben und bevorzugt mit diesem über einen an sich bekannte Standard-Kupplung verbunden. Die Kanüle 31 weist einen Kanal 5 auf, durch den die verschiedenen für die Durchführung eines Operationsvorgangs benötigten Instrumente, wie Sektionsinstrumente, Scheren, HF-Instrumente usw. zum distalen Ende 2 durchgeführt werden können.

Ferner weist die Kanüle 31 zwei nicht im einzelnen dargestellte Kanäle auf, von denen der eine ein Zufuhrkanal für eine Flüssigkeit mit einer distal angeordneten Fluid-Austrittsöffnung, und der andere ein Absaugkanal mit einer distal angeordneten Fluid-Ansaugöffnung ist.

Diese beiden Kanäle können insbesondere auf der gegenüberliegenden Seite des Kanals 5 angeordnet sein.

Der Zufuhrkanal und der Absaugkanal sind über zwei Luer-Anschlüsse 61 und 62 mit einer Pumpeneinrichtung verbunden, die beispielsweise eine Pumpe vom Typ Sero-Konditionierpumpe nach Manhes sein kann.

Ferner ist eine nicht dargestellte elektronische Steuereinheit vorgesehen, die die Pumpeneinrichtung steuert. Die Steuereinheit steuert insbesondere den Druck, mit dem die Flüssigkeit, die insbesondere Wasser - gegebenenfalls mit Zusätzen - sein kann, aus der distal angeordneten - nicht näher dargestellten - Austrittsöffnung austritt, sowie den Druck, mit dem Flüssigkeit aus der das distale Ende des Instruments umgebenden Flüssigkeitsblase angesaugt wird. In Abhängigkeit von der Dichte und der Konsistenz des zu trennenden Gewebes kann der Druck, mit dem die Flüssigkeit austritt, typischerweise zwischen 10⁴ (0,1 bar) und 10⁵ Pascal (1 bar) liegen. Diese Drücke sind deutlich höher als die Drücke, die im Bereich der Endoskopie und insbesondere der Laparoskopie zum kontinuierlichen Spülen verwendet werden, wie dies beispielsweise in der US-PS 5 392 765 beschrieben ist.

Erfindungsgemäß wird durch die nicht näher dargestellte Pumpeneinheit, die zum Spülen und Absaugen dient, eine Wasserblase geschaffen, die das Gewebe längs natürlicher Spaltungsebenen aufweitet. Diese "Wasserblase" macht es möglich, dass sich der Instrumentenverbund (Laparoskop, Kanüle und Instrument) in das Gewebe hinein vorwärts bewegen kann.

Der Operateur sieht dank der unter ca. 12° schräg stehenden Optik laufend das verwendete Instrument, das insbesondere etwas länger sein kann als der Kanal, in das das Instrument eingesetzt ist.

Vorstehend ist die Erfindung anhand eines Ausführungsbeispiels beschrieben worden. Selbstverständlich sind innerhalb des allgemeinen Erfindungsgedankes die verschiedensten Modifikationen möglich:

So ist es möglich, die distale Austrittsöffnung des Zufuhrkanals als Düse auszubilden, um die Effizienz des erfindungsgemäß ausgeführten "Wasserstrahl-Schneidens" bzw. .,-Trennens" zu erhöhen.

Weiterhin sind die angegebenen Druckwerte nicht einschränkend; im Einzelfall ist ein Unterschreiten und ein Überschreiten möglich.

## Patentansprüche

1. Chirurgisches Instrumentensystem insbesondere zur Verwendung bei nicht endoskopischen Operationen, mit einem Operationsinstrument, das einen Schaft aufweist, in dem ein Zufuhrkanal (3) mit einer distal angeordneten Fluid-Austrittsöffnung, und ein Absaugkanal mit einer distal angeordneten Fluid-Ansaugöffnung vorgesehen sind,
**dadurch gekennzeichnet, dass** eine Pumpeneinrichtung vorgesehen ist, die eine Flüssigkeit mit einem derartigen Druck in den Zufuhrkanal pumpt, dass das Gewebe vor dem distalen Ende (2) des Operationsinstruments durch die aus der Austrittsöffnung austretende Flüssigkeit längs natürlicher Spaltungsebenen getrennt wird,
wobei die Flüssigkeit aus der das distale Ende (2) des Operationsinstruments umgebenden Flüssigkeitsblase durch den Absaugkanal zum proximalen Ende des Operationsinstruments gepumpt wird.

2. Instrumentensystem nach Anspruch 1,
**dadurch gekennzeichnet, dass** in dem Schaft wenigstens ein Operationskanal vorgesehen ist, der das Einführen von Behandlungsinstrumenten, wie z.B. Instrumenten zum Schneiden, Spreizen oder Koagulieren ermöglicht.

3. Instrumentensystem nach Anspruch 1 oder 2,
**dadurch gekennzeichnet, dass** der Schaft eine Endoskopoptik aufweist, oder dass er Schaft einen Kanal aufweist, in den eine Endoskopoptik einsetzbar ist.

4. Instrumentensystem nach Anspruch 3,
**dadurch gekennzeichnet, dass** das Objektiv der Endoskopoptik eine Blickrichtung bzw. eine objektseitige optische Achse hat, die einen kleinen Winkel mit der Längsachse des Schaftes einschließt.

5. Instrumentensystem nach Anspruch 4,
**dadurch gekennzeichnet, dass** der kleine Winkel ca. 12° beträgt.

6. Instrumentensystem nach einem der Ansprüche 1 bis 5,
**dadurch gekennzeichnet, dass** der Schaft den Grundaufbau eines Laparoskops (1) mit seitlich versetzten Einblick hat, und dass in den Instrumentenkanal des Laparoskops (1) eine Doppelkanüle (31) eingesetzt ist, in der der Zufuhrkanal und der Absaugkanal vorgesehen sind.

7. Instrumentensystem nach Anspruch 6,
**dadurch gekennzeichnet, dass** der Instrumentenkanal zentral in dem Schaft angeordnet ist.

8. Instrumentensystem nach einem der Ansprüche 6 und 7,
**dadurch gekennzeichnet, dass** der Operationskanal zentral in der Doppelkanüle (31) vorgesehen ist.

9. Instrumentensystem nach einem der Ansprüche 6 bis 8,
**dadurch gekennzeichnet, dass** die Doppelkanüle (31) zwei Anschlüsse für die Pumpeneinrichtung aufweist.

10. Instrumentensystem nach Anspruch 9,
**dadurch gekennzeichnet, dass** die Anschlüsse (61, 62) Luer-Lock- Anschlüsse sind.

11. Instrumentensystem nach einem der Ansprüche 1 bis 10,
**dadurch gekennzeichnet, dass** die Pumpeneinrichtung eine Konditionierpumpe nach Manhes ist.

12. Instrumentensystem nach einem der Ansprüche 1 bis 11,
**dadurch gekennzeichnet, dass** eine elektronische Steuereinheit vorgesehen ist, die die Pumpeneinrichtung derart steuert, dass der Druck, mit dem die Flüssigkeit austritt, der Konsistenz bzw. der Dichte der zu trennenden Gewebeschichten etc. angepasst ist.

13. Instrumentensystem nach einem der Ansprüche 1 bis 12,
**dadurch gekennzeichnet, dass** die Flüssigkeit Wasser ist.

14. Instrumentensystem nach einem der Ansprüche 1 bis 13,
**dadurch gekennzeichnet, dass** der Druck, mit dem die Flüssigkeit austritt, zwischen ca. 10⁴ Pascal (0,1 bar) und ca. 10⁵ Pascal (1 bar) beträgt.

## Claims

1. Surgical instrument system, particularly for application in non-endoscopic operations, comprising a surgical instrument including a shaft in which a supply duct (3) with a distally disposed fluid discharge opening and an exhaust duct with a distally disposed fluid intake opening are provided,
**characterised in that** a pumping means is provided which pumps a liquid into said supply duct at such a pressure that the tissue in front of the distal end (2) of the operating instrument will be severed by the liquid discharged from said discharge opening along natural cleaving planes,
wherein the liquid is pumped from the liquid bubble surrounding the distal end (2) of said surgical instrument and through said exhaust duct towards the proximal end of the operating instrument.

2. Instrument system according to Claim 1,
**characterised in that** at least one operating duct is provided in said shaft, which permits the introduction of operating instruments such as instruments for cutting, spreading or coagulation.

3. Instrument system according to Claim 1 or 2,
**characterised in that** said shaft comprises an optical endoscope system or that said shaft includes a duct into which an optical endoscope system may be introduced.

4. Instrument system according to Claim 3,
**characterised in that** the lens of said optical endoscope system presents a viewing direction or an optical axis on the object side, respectively, that forms a small angle relative to the longitudinal axis of said shaft.

5. Instrument system according to Claim 4,
**characterised in that** said small angle amounts to 12° approximately.

6. Instrument system according to any of the Claims 1 to 5,
**characterised in that** said shaft presents the fundamental structure of a laparoscope (1) with laterally offset observation opening, and that a double hollow needle (31) is introduced into said instrument duct of the laparoscope, wherein said supply duct and said exhaust duct are provided.

7. Instrument system according to Claim 6,
**characterised in that** said instrument duct is disposed in a central position in said shaft.

8. Instrument system according to any of the Claims 6 and 7,
**characterised in that** said operating duct is provided in a central position in said double hollow needle (31).

9. Instrument system according to any of the Claims 6 to 8,
**characterised in that** said double hollow needle (31) comprises two connectors for said pumping means.

10. Instrument system according to Claim 9,
**characterised in that** said connectors (61, 62) are Luer lock connectors.

11. Instrument system according to any of the Claims 1 to 10,
**characterised in that** said pumping means is a conditioning pump according to Manhes.

12. Instrument system according to any of the Claims 1 to 11,
**characterised in that** an electronic controller is provided for controlling said pumping means in such a way that the pressure at which the liquid is discharged is adapted to the consistence or the density of the tissue layers to be severed, etc.

13. Instrument system according to any of the Claims 1 to 12,
**characterised in that** said liquid is water.

14. Instrument system according to any of the Claims 1 to 13,
**characterised in that** the pressure at which the liquid is discharged, is at a level in the range between roughly 10⁴ Pascal (0.1 bar) and 105 Pascal (0.1 bar).

## Revendications

1. Système d'instrument chirurgical, en particulier pour l'emploi dans des opérations non endoscopiques, comprenant un instrument chirurgical renfermant une tige, dans laquelle sont disposé un canal d'amenée (3) à un orifice de sortie de fluide, disposé du côté distal, et un canal d'évacuation à une ouverture d'admission de fluide, disposée du côté distal,
**caractérisé en ce qu'**un moyen de pompe est disposé, qui pompe un liquide dans ledit canal d'amenée à une telle pression, que le tissu avant l'extrémité distale (2) de l'instrument chirurgical soit séparé moyennant le liquide sortant dudit orifice de sortie, le long des plans de fendage naturels,
dans lequel le liquide est pompé des la bulle de liquide, qui entoure l'extrémité distale (2) dudit instrument chirurgical et par ledit canal d'évacuation vers l'extrémité proximale dudit instrument chirurgical.

2. Système d'instrument selon la revendication 1,
**caractérisé en ce qu'**au moins un canal opératoire est formé dans ladite tige, qui permet l'introduction des instruments chirurgicaux, par exemple des instruments à couper, à étendre ou à coaguler.

3. Système d'instrument selon la revendication 1 ou 2,
**caractérisé en ce que** ladite tige comprend un système optique d'endoscope ou que ladite tige renferme un canal dans lequel on peut introduire un système optique d'endoscope.

4. Système d'instrument selon la revendication 3,
**caractérisé en ce que** l'objectif dudit système optique d'endoscope présente une direction de visée ou respectivement un axe optique du côté d'objet, qui s'étend à un petit angle relatif à l'axe longitudinale de ladite tige.

5. Système d'instrument selon la revendication 4,
**caractérisé en ce que** ledit petit angle correspond à 12° environ.

6. Système d'instrument selon une quelconque des revendications 1 à 5,
**caractérisé en ce que** ladite tige a la structure fondamentale d'un laparoscope (1) à une ouverture d'observation latéralement désaxée, et **en ce qu'**une canule double (31) est introduite dans ledit canal d'instrument du laparoscope, dans lequel sont formé ledit canal d'amenée et ledit canal d'évacuation.

7. Système d'instrument selon la revendication 6,
**caractérisé en ce que** ledit canal d'instrument est disposé à une position centrale dans ladite tige.

8. Système d'instrument selon une quelconque des revendications 6 et 7,
**caractérisé en ce que** ledit canal opératoire est disposé à une position centrale dans ladite canule double (31).

9. Système d'instrument selon une quelconque des revendications 6 à 8,
**caractérisé en ce que** ladite canule double (31) comprend deux raccords pour ledit moyen de pompe.

10. Système d'instrument selon la revendication 9,
**caractérisé en ce que** lesdits raccords (61, 62) sont des raccords du type Luer Lock.

11. Système d'instrument selon une quelconque des revendications 1 à 10,
**caractérisé en ce que** ledit moyen de pompe est une pompe de conditionnement selon Manhes.

12. Système d'instrument selon une quelconque des revendications 1 à 11,
**caractérisé en ce qu'**une unité de commande électronique est pourvue à commander ledit moyen de pompe de façon que la pression, sous laquelle ledit liquide sort, soit adaptée à la consistance ou la densité des couches de tissu à couper, etc.

13. Système d'instrument selon une quelconque des revendications 1 à 12,
**caractérisé en ce que** ledit liquide est de l'eau.

14. Système d'instrument selon une quelconque des revendications 1 à 13,
**caractérisé en ce que** la pression de sortie du liquide est à un niveau dans la gamme entre 10⁴ Pascal (0.1 bar) environ et 105 Pascal (0.1 bar) environ.
